(19) Europäisches Patentamt
European Patent Office
Office européen des brevets

(11) Publication number: **0 324 520 A2**

(12) # EUROPEAN PATENT APPLICATION

(21) Application number: 89200028.2

(22) Date of filing: 06.01.89

(51) Int. Cl.4: C07D 239/84 , C07D 239/94 , C07D 239/70 , A61K 31/505

(30) Priority: 14.01.88 US 143895

(43) Date of publication of application: 19.07.89 Bulletin 89/29

(84) Designated Contracting States: CH DE FR GB IT LI NL

(71) Applicant: MERCK & CO. INC.
126, East Lincoln Av…nue P.O. Box 2000
Rahway New Jersey 07065-0900(US)

(72) Inventor: Wu, Mu T.
35 Lance Drive
Clark New Jersey 07066(US)
Inventor: Tolman, Richard L.
29 Upper Warren Way
Warren New Jersey 07060(US)
Inventor: Maccoss, Malcolm
48 Rose Court
Freehold New Jersey 08828(US)

(74) Representative: Hesketh, Alan, Dr.
European Patent Department Merck & Co., Inc. Terlings Park Eastwick Road
Harlow Essex, CM20 2QR(GB)

(54) **Alkyl-piperazinyl-5-6-alkylenepyrimidines.**

(57) Compounds of formula (I)

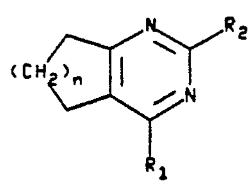

(I)

wherein one of $R_1$ or $R_2$ is piperazinyl or substituted piperazinyl and the other is alkyl or hydrogen possess oral hypoglycemic activity with the ability to lower blood sugar and are thus useful in the treatment of type II diabetes with associated insulin resistance.

EP 0 324 520 A2

# ALKYL-PIPERAZINYL-5,6-ALKYLENEPYRIMIDINES

## BACKGROUND OF THE INVENTION

Diabetes is a condition characterized by abnormal insulin secretion and a variety of metabolic and vascular manifestations reflected in a tendency toward inappropriately elevated blood glucose levels and which if left poorly treated or untreated can result in accelerated, nonspecific atherosclerosis, neuropathy and thickened capillary lamina causing renal and retinal impairment. Diabetes is characterized as being insulin dependent (Type I) and non-insulin dependent (Type II). Type I diabetes is due to damage and eventual loss of the β-cells of the pancreatic islets of Langerhans with a resulting loss of insulin production. Type II diabetics secrete insulin, however, the insulin is somehow not properly or effectively utilized in the metabolism of blood sugars and glucose accumulates in the blood to above normal levels. This condition is termed insulin resistance.

With the certainty of serious complications resulting from high glucose levels in poorly controlled or uncontrolled diabetics, means to lower blood glucose have been research goals for a considerable period of time. With Type I diabetes glucose control can only be achieved with daily insulin injections. With Type II diabetes glucose control can be effected from a combination of diet and drugs which lower glucose levels. The currently available oral hypoglycemic agents are not completely satisfactory since they may not offer complete blood glucose control or may provide a variety of undesirable side effects or they may elevate insulin concentrations to undesirable and dangerous levels. Thus, the search for improved oral hypoglycemic agents is a continuing one.

U.K. Patent 2,119,368 discloses compounds of hypoglycemic activity which are substituted thiopyranopyrimidine compounds of the formula:

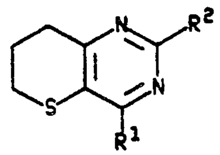

wherein $R^1$ is an amino, methylamino, hydroxyethylamino, pyrrolidino, morpholino, piperazino or N-substituted piperazino group and $R^2$ is an amino, methylamino, dimethylamino, piperazino, N-substituted piperazino, pyrollidino, piperidino or morpholino group.

These compounds lacking alkyl substitution on the heterocycle rings, are structurally distinct from those in the instant invention.

U.S. Patent 4,352,928 and a corresponding publication Sekiya, Eur. J. Med. Chem., 15, 317 (1980) disclose 2-piperazino-5,6-polymethylene pyrimidines of the structural formula:

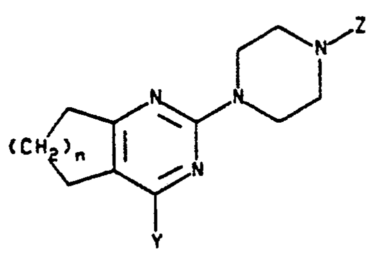

where Y, Z and n are defined in the specification of the above patent. These compounds do not contain a hydrogen, alkyl or substituted alkyl group in the 2 or 4 position of the pyrimidine ring and are thus structurally distinct from the compounds of the instant invention.

## DESCRIPTION OF THE INVENTION

The alkyl-piperazinyl-5,6-alkylenepyrimidines of the instant invention are compounds of hypoglycemic

activity and structural formula (I):

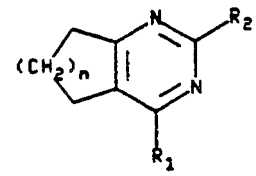

(I)

wherein
n is 1 or 2;

**$R_1$ is** 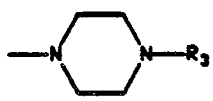 **or $C_{1-6}$alkyl or H;**

**$R_2$ is** 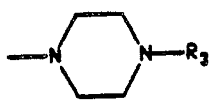 **or $C_{1-6}$alkyl or H;**

**provided that one of $R_1$ or $R_2$ is** 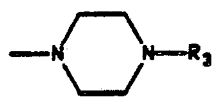 **and the other is $C_{1-6}$alkyl or H;**

$R_3$ is H, $C_{1-6}$alkyl, $C_{2-6}$alkenyl,
$C_{2-6}$alkynyl, phenyl$C_{1-6}$alkyl.

The alkyl, alkenyl and alkynyl groups of the present invention may either be in a straight or branched configuration.

One embodiment of the present invention are compounds of formula (I) wherein $R_1$ is $C_{1-6}$alkyl or H, and $R_2$ is

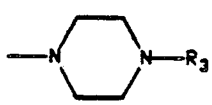

In one class of this embodiment are those compounds wherein n is 2, and $R_1$ is $CH_3$. Exemplifying this class are:

a: 5,6,7,8-Tetrahydro-4-methyl-2-(1-methylpiperazinyl)quinazoline
b: 5,6,7,8-Tetrahydro-4-methyl-2-(1-ethylpiperazinyl)quinazoline.
c: 5,6,7,8-Tetrahydro-4-methyl-2-(1-benzylpiperazinyl)-quinazoline.
d: 5,6,7,8-Tetrahydro-4-methyl-2-(piperazinyl)quinazoline.

In a second class are those compounds wherein n = 1 and $R_1$ is $CH_3$.

In a second embodiment are those compounds of formula (I) wherein $R_1$ is

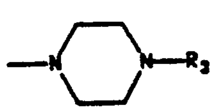

and $R_2$ is $C_{1-6}$alkyl or H.

The compounds of formula (I) wherein n = 2, $R_1$ = $C_{1-6}$alkyl and $R_2$ is

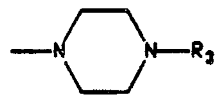

are prepared according to the following scheme:

## SCHEME 1

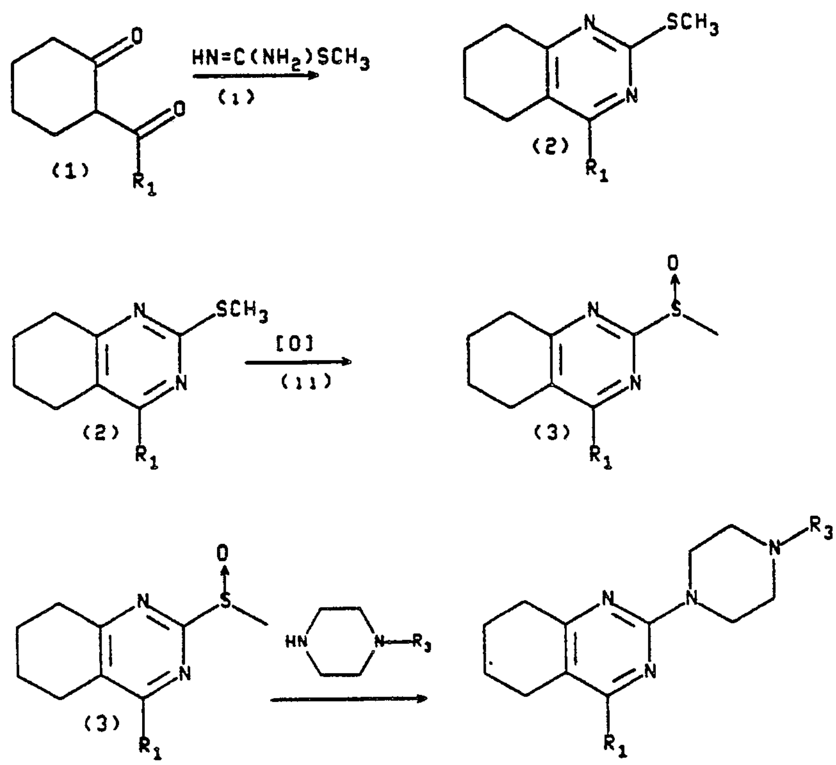


In step (i) of Scheme (1) a 2-alkanoylcyclohexanone or a 2-formylcyclohexanone is mixed with a base such as an alkali metal hydroxide in an alcoholic solvent; to this mixture, at room temperature, is added 2-methyl-2-thiopseudourea sulfate, and the reaction mixture stirred for about 18 hours. The reaction mixture is then poured onto ice-water, acidified with an acid, such as acetic acid, and the product oil collected.

In step (ii) the methylthio group of (2) is oxidized to a methylsulfinyl group to form compound (3). An oxidizing agent such as m-chloroperbenzoic acid in a solvent such as chloroform is added to a solution of compound (2) in a solvent such as chloroform at a temperature of about -10°C, for 3 to 24 hours, preferably 16 hours. Preferably equal numbers of moles of oxidizing agent and compound (2) are used.

The 4′ substituted piperazino group is attached to the 2-position of the pyrimidine ring by substitution of the methylsulfinyl group. A mixture of compound (3) and a N-substituted piperazine is heated under an inert atmosphere, preferably nitrogen at reflux for about 24 hours. The piperazino reagent is used in excess; preferably 4 equivalents of the piperazine compound are employed. The product is isolated from the reaction mixture using standard techniques.

A compound wherein $R_3$ is H, is formed from the corresponding structure wherein $R_3$ = benzyl by substitution of benzyl by H. A compound wherein $R_3$ = benzyl is combined with a mixture of about 10% Pd/C in absolute alcohol, preferably absolute ethanol. The mixture is hydrogenated at about 40 psi at approximately room temperature for about 19 hours. The product is isolated using standard techniques.

The compounds of formula (I) wherein n = 1, $R_1$ = $C_{1-6}$alkyl or H and $R_2$ is

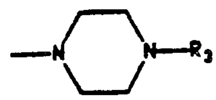

are prepared according to the procedure of Scheme 1 and the conditions expressed above but substituting 2-alkanoylcyclopentanone or 2-formylcyclopentanone for their cyclohexanone analogs.

The compounds of formula (I) wherein n = 2,

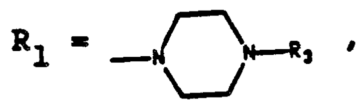

and $R_2$ = $C_{1-6}$alkyl or H are prepared according to Scheme 2:

**SCHEME 2**

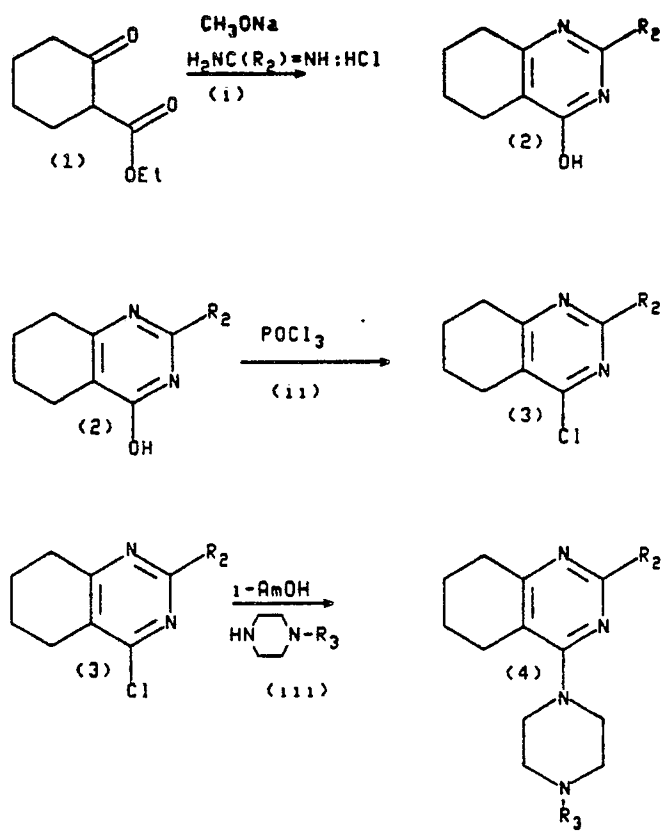


In Step (i) of Scheme 2 an amidine is added to an equivalent amount of sodium methoxide and, after removal of the precipitated NaCl, the solution is added to an equivalent amount of ethyl 2- cyclohexanonecarboxylate. The amidine can be formed by addition of ammonia to a nitrile.

In Step (ii) the 4-hydroxy quinazoline product is suspended in phosphorous oxychloride and refluxed for about 3 hours.

The 4′-substituted piperazino group is attached to the 4-position of the quinazoline ring by substitution of the chloro group. Where $R_3$ = H, the piperazino group is substituted with a <u>tert</u>-butoxycarbonyl (t-BOC) blocking group, which is later removed by reaction with trifluoroacetic acid.

The compounds of formula (I) wherein n = 1,

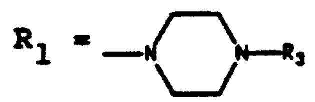

and $R_2$ = $C_{1-6}$alkyl or H are prepared according to the procedure of Scheme 2 and the conditions expressed above, but substituting ethyl 2-cyclopentanonecarboxylate for ethyl 2-cyclohexanone carboxylate.

The compounds of this invention are all readily adapted to therapeutic use as oral hypoglycemic agents. These compounds lower the blood sugar levels of diabetic subjects to a statistically significant degree. For instance, a typical and preferred agent of the present invention 5,6,7,8-tetrahydro-4-methyl-2-(1-methylpiperazinyl) quinazoline, has been found to consistently lower blood sugar levels and improve glucose tolerance in either fasted or fed diabetic (i.e., hyperglycemic) mice to a statistically significant degree when given by the oral route of administration at dose levels ranging from 1 mg/kg to 100mg/kg, respectively, without showing any toxic side effects. The other compounds of this invention also produce similar results. In general, these compounds are ordinarily administered at dosage levels ranging from about 1 mg to about 100 mg per kg of body weight per day, although variations will necessarily occur depending upon the condition and individual response of the subject being treated and the particular type of oral pharmaceutical formulation chosen.

Administration over time to obese, insulin resistant mice,resulted in a significant reduction of glucose in a glucose tolerance test.

In connection with the use of the compounds of this invention for the treatment of diabetic subjects, it is to be noted that they may be administered either alone or in combination with pharmaceutically acceptable carriers and that such administration can be carried out in both single and multiple dosages. More particularly, the novel compounds of the invention can be administered in awide variety of different dosage forms, i.e., they may be combined with various pharmaceutically acceptable inert carriers in the forms of tablets, capsules, lozenges, troches, hard candies, powders, aqueous suspension, elixirs, syrups and the like. Such carriers include diluents or fillers, sterile aqueous media and various non-toxic organic solvents, etc. Moreover, such oral pharmaceutical compositions can be suitably sweetened and/or flavored by means of various agents of the type commonly employed for just such a purpose. In general, the therapeutically-effective compounds of this invention are present in such dosage forms at concentration levels ranging from about 0.5% to about 90% by weight of the total composition, i.e., in amounts which are sufficient to provide the desired unit dosage.

For purposes of oral administration, tablets containing various excipients such as sodium citrate, calcium carbonate and dicalcium phosphate may be employed along with various disintegrants such as starch and preferably potato or tapioca starch, alginic acid and certain complex silicates, together with binding agents such as polyvinylpyrrolidone, gelatin and acacia. Additionally, lubricating agents such as magnesium stearate, sodium lauryl sulfate and talc are often very useful for tabletting purposes. Solid compositions of a similar type may also be employed as fillers in soft and hard-filled gelatin capsules; preferred materials in this connection would also include the high molecular weight polyethylene glycols. Whe aqueous suspensions and/or elixirs are desired for oral administration, the essential active ingredient therein may be combined with various sweetening or flavoring agents, coloring matter or dyes and, if so desired, emulsifying and/or suspending agents as well, together with such diluents as water, ethanol, propylene glycol, glycerin and various like combinations thereof.

The activity of the compounds of the present invention, as hypoglycemic agents, is determined by their ability to lower blood sugar levels in the fasted or fed hyperglycemic mouse when tested therein for such purposes according to the procedures described by Saperstein et al. as submitted to the journal Diabetes and summarized as follows: Genetically obese mice (ob/ob) were fasted overnight. The compounds were administered orally via a stomach tube and each mouse serially bled from the orbital sinus at various times and the blood samples were analyzed for blood glucose. When the effects of the compounds on the blood glucose levels were to be determined, glucose was administered orally at a rate of 2 g per kg. 30 minutes after administration of the test compound. Glucose in the blood was determined by the potassium ferricyanide-potassium ferrocyanide oxidation reaction auto analyzer.

The latter method measures directly the amount of glucose in the blood at any given time and from this, the maximum percent decrease in blood sugar can be readily calculated and reported as hypoglycemic activity per se. In this way, the present compounds are shown to markedly improve glucose tolerance of non-anesthetized hyperglycemic mice when administered to them at dose levels as low as 10 mg/kg orally and lower fasting blood glucose levels when administered at dose levels as low as 30 mg/kg orally.

The instant invention is further described by the following examples which are intended to be merely descriptive and should not be construed as limitative of the invention.

## EXAMPLE 1

### Preparation of 5,6,7,8-Tetrahydro-4-methyl-2-(1-methylpiperazinyl) quinazoline

#### a: 5,6,7,8-Tetrahydro-4-methyl-2-methyl-thioquinazoline

14 g (0.1 mole) of 2-acetylcyclohexanone was added to 120 ml of methanolic solution containing 11.2 g (0.2 mole) of potassium hydroxide at room temperature while stirring. 27.8 g (0.1 mole) of 2-methyl-2-thiopseudourea sulfate was then added to the mixture and the stirring was continued for 18 hours. The reaction mixture was then poured into ice-water, and the mixture was made acidic with acetic acid. The oil which separated was collected by decantation and triturated with ice water to give 5 g of 5,6,7,8-tetrahydro-4-methyl-2-methylthioquinazoline, m.p. 38-40 °C. The product was recrystallized from iPrOH-$H_2$0, m.p. 43-44 °C; MS (EI): m/e 194 (M+). 200 MHz NMR ($CDCl_3$) w: 1.80 (4H, m), 2.34 (3H, s), 2.52 (3H, s), 2.57 (2H, m), 2.76 (2H, s).

| Anal. Calcd. for $C_{10}H_{14}N_2S$: | | | | | | |
|---|---|---|---|---|---|---|
| | C, | 61.84; | H, | 7.27; | N, | 14.42. |
| Found: | C, | 61.78; | H, | 7.10; | N, | 14.50. |

#### b: 5,6,7,8-Tetrahydro-4-methyl-2-methylsulfinylquinazoline

A solution of 80% m-chloroperbenzoic acid (1.56 g, 0.00721 mole) in 30 ml of chloroform was added to a solution of 1.4 g (0.00721 mole) of 5,6,7,8-tetrahydro-4-methyl-2-methylthioquinazoline in 30 ml of chloroform at -10 °C. The reaction mixture was kept at -10 °C for 3 hours, and allowed to stand at room temperature overnight. After the mixture had been washed with 5% $NaHCO_3$ solution, the chloroform solution was dried over $MgSO_4$, and concentrated. The residue solidified on cooling, weighing 1.41 g m.p. 85-95 °C; TCL: single spot, Rf 0.65, EtoAC: nBuOH: $H_2O$; HOAc (1:1:1:1); MS (EI): m/e 210 ($M^+$).

#### 5,6,7,8-Tetrahydro-4-methyl-2-(1-methyl piperazinyl) quinazoline

A mixture of 631 mg (0.003 mole) of 5,6,7,8-tetrahydro-4-methyl-2-methylsulfinylquinazoline and 1.2 g (0.012 mole) of N-methylpiperazine was heated under nitrogen at reflux for 24 hours. Water was added to the mixture, and the mixture extracted with chloroform, washed with 5% sodium bicarbonate, water and treated with Darco (activated carbon). The solution was then dried over $MgSO_4$, filtered, and concentrated under reduced pressure to give 655 mg of the titled compound. A portion (200 mg) of the free base thus obtained was converted into its maleate in methanol and recrystallized from methanol to give 5,6,7,8-tetrahydro-4-methyl-2-(1-methyl-piperazinyl) quinazoline maleate, m.p. 178-180 °C. Purification by Dowex 1-2X ($OH^-$), gave m.p. 186-187 °C; MS(EI): m/e 246 ($M^+$). 200 MHz NMR ($D_2O$) w: 1.58 (4H, m), 2.06 (3H, s), 2.32 (2H, m), 2.44 (2H, m), 2.68 (3H, s), 3.06 (4H, m), 4.12 (4H, m), 6.08 (4H, s).

| Anal. Calcd. for $C_{14}H_{22}N_4{}^{\prime\prime}C_4H_4O_4$: | | | | | | |
|---|---|---|---|---|---|---|
| | C, | 59.65; | H, | 7.23; | N, | 15.46. |
| Found: | C, | 59.59; | H, | 7.24; | N, | 15.37. |

## EXAMPLE 2

Preparation of 5,6,7,8-tetrahydro-4-methyl-2-(1-ethylpiperazinyl) quinazoline

Steps (a) and (b) of Example 1 are repeated as described above.

c: 5,6,7,8-Tetrahydro-4-methyl-2-(1-ethylpiperazinyl)-quinazoline

In the same manner as described in Step 1C, 5,6,7,8-tetrahydro-4-methyl-2-methylsulfinylquinazoline (720 mg, 0.003425 mole) and N-ethylpiperazine (1.7 g, 0.01489 mole) were heated to afford 5,6,7,8-tetrahydro-4-methyl-2-(1-ethylpiperazinyl) quinazoline (677 mg). Maleate: Recrystallized from isopropanol, m.p. 183-154.5° C; MS (EI): m/e 260 ($M^+$). 300 MHz NMR ($D_2O$) w: 1.37 (3H, t), 1.79 (4H, m), 2.32 (3H, s), 2.55 (2H, m), 2.68 (2H, m), 3.10 (4H, m), 3.27 (2H, q), 3.67 (4H, m), 6.29 (2H, s)

| | | | | | | |
|---|---|---|---|---|---|---|
| Anal. Calcd. for $C_{15}H_{24}N_4$ " $C_4H_4O$: | C, | 60.62; | H, | 7.50; | N, | 14.88. |
| Found: | C, | 60.45; | H, | 6.96; | N, | 14.37. |

## EXAMPLE 3

Preparation of 5,6,7,8-tetrahydro-4-methyl-2-(1-benzylpiperazinyl) quinazoline

Steps (a) and (b) of Example 1 are repeated as described above.

c: 5,6,7,8-Tetrahydro-4-methyl-2-(1-benzylpiperazinyl) quinazoline

In the same manner as described in Step 1(c), 5,6,7,8-tetrahydro-4-methyl-2-methylsulfinylquinazoline (1.4 g, 0.00666 mole) and 1-benzylpiperazine (4 g, 0.0227 mole) were heated at 180° C for 10 hours to obtain 5,6,7,8-tetrahydro-4- methyl-2-(1-benzylpiperazinyl) quinazoline as an oil.
Maleate: m.p. 180-182° C; MS (EI): m/e 322 ($M^+$). 300 MHz NMR (1 eq. DCl in $D_2O$) w: 1.83 (4H, m), 2.49 (3H, s), 2.61 (2H, m), 2.84 (2H, m), 3.3-3.7 (8H, m), 4.45 (2H, s), 6.41 (2H, s), 7.55 (5H, m).

## EXAMPLE 4

Preparation of 5,6,7,8-tetrahydro-4-methyl-2-piperazinylquinazoline

A mixture of 5,6,7,8-tetrahydro-4-methyl-2-(1-benzylpiperazinyl) quinazoline (750 mg, 0.00233 mole) and 10% Pd/C (2 g) in absolute ethanol (40 ml) was hydrogenated at 40 psi with rocking at room temperature. After 19 hours, the reaction mixture was filtered and concentrated in vacuo to leave 5,6,7,8-tetrahydro-4-methyl-2-piperazinylquinazoline. Maleate: m.p. 174-175° C; MS (EI): m/e 232 ($M^+$). 200 MHz NMR ($CDCl_3/CD_3OD$) w: 1.61 (4H, m), 2.10 (3H, s), 2.36 (2H, m), 2.48 (2H, m), 3.03 (4H, m), 3.85 (4H, m), 6.10 (4H, s).

## EXAMPLES 5-8

The following compounds are prepared following the procedures of Examples 1-4 but substituting 2-acetylpentanone in place of 2-acetylhexanone.

Example 5: 2-(1-methylpiperazinyl)-4-methyl-5,6-trimethylenepyrimidine.

Example 6: 2-(1-ethylpiperazinyl)-4-methyl-5,6-trimethylenepyrimidine

Example 7: 2-(1-benzylpiperazinyl)-4-methyl-5,6-trimethylenepyrimidine.

Example 8: 2-piperazinyl-4-methyl-5,6-trimethylenepyrimidine.

## EXAMPLE 9

Preparation of 5,6,7,8-tetrahydro-2-methyl-4-piperazinylquinazoline dihydrochloride

Acetamidine hydrochloride is added to an equivalent amount of 2N sodium methodoxide in methanol. After 5-10 minutes, when sodium chloride precipitation is complete, the solution of amidine is filtered directly into an equivalent of ethyl 2-cyclohexanonecarboxylate. After the reaction mixture is allowed to stand overnight, 5,6,7,8-tetrahydro-2-methyl-4-hydroxyquinazoline is isolated by filtration. The product is then suspended in phosphorous oxychloride and refluxed for 3 hours. After cooling, the reaction mixture is poured onto ice followed by extraction with methylene chloride. The extract is washed with water and dried over anhydrous magnesium sulfate. After removal of the solvent, the residue is chromatographed on silica gel, eluted with chloroform to give 5,6,7,8-tetrahydro-2-methyl-4-chloroquinazoline. A solution (1 mmol/4 ml.) of the above chloro compound in isoamyl alcohol is added dropwise to a solution (1 mmol/4 ml) of N-(tert-butoxycarbonyl)- piperazine in isoamyl alcohol at 120° C. After 4 hours the reaction mixture is concentrated to dryness under reduced pressure. Following the removal of the tert-butoxycarbonyl group with trifluoroacetic acid, the product in aqueous solution is passed through a column of Dowex-1 ion exchange resin on the hydroxide ion cycle. After the aqueous solution is concentrated to dryness, the residue is converted to the hydrochloride salt with 2N HCl. The product is crystallized from ethanol-ether to yield 5,6,7,8-tetrahydro-2-methyl-4-piperazinylquinazoline dihydrochloride.

**Claims**

1. A compound of structural formula (I):

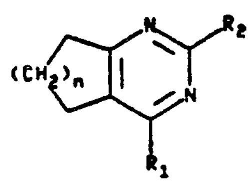

(I)

**wherein**

**n is 1 or 2;**

**$R_1$ is** 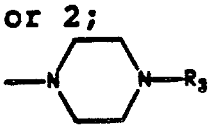 **or $C_{1-6}$alkyl or H;**

**$R_2$ is** 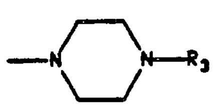 **or $C_{1-6}$alkyl or H;**

provided that one of $R_1$ or $R_2$ is

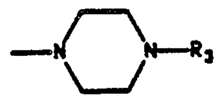

and the other is $C_{1-6}$alkyl or H;
$R_3$ is H, $C_{1-6}$alkyl, $C_{2-6}$alkenyl,
$C_{2-6}$alkynyl, phenyl$C_{1-6}$alkyl;
and the pharmaceutically acceptable salts thereof.

2. A compound of Claim 1 wherein: $R_1$ is $C_{1-6}$alkyl;

$R_2$ is 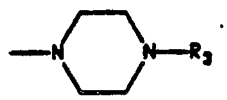

3. A compound of Claim 2 wherein:
n is 2; and
$R_1$ is $CH_3$.

4. A compound of Claim 3 selected from:

a. 5,6,7,8-Tetrahydro-4-methyl-2-(1-methylpiperazinyl) quinazoline.
b. 5,6,7,8-Tetrahydro-4-methyl-2-(1-ethylpiperazinyl) quinazoline.
c. 5,6,7,8-Tetrahydro-4-methyl-2-(1-benzylpiperazinyl) quinazoline.
d. 5,6,7,8-Tetrahydro-4-methyl-2-(piperazinyl) quinazoline.

5. A compound of Claim 2 wherein n is 1 and $R_1$ is $CH_3$.

6. A hypoglycemic pharmaceutical composition comprising a nontoxic therapeutically effective amount of a compound of Claim 1 and a pharmaceutically acceptable carrier.

7. The use of a compound of Claim 1 for the preparation of a medicament useful for the treatment of diabetes or obesity with associated insulin resistance.

8. The use of a compound of Claim 5 for the preparation of a medicament useful for the treatment of diabetes or obesity with associated insulin resistance.

9. A process for the preparation of a compound of Claim 2 which comprises treating a compound of formula:

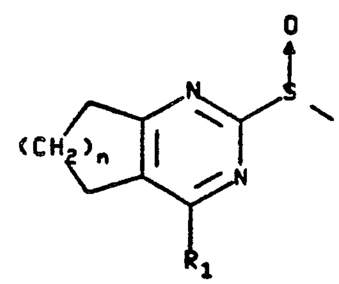

with